(19) Europäisches Patentamt
European Patent Office
Office européen des brevets

(11) **EP 1 740 111 B1**

(12) **EUROPÄISCHE PATENTSCHRIFT**

(45) Veröffentlichungstag und Bekanntmachung des Hinweises auf die Patenterteilung:
**05.08.2009 Patentblatt 2009/32**

(21) Anmeldenummer: **04729808.8**

(22) Anmeldetag: **28.04.2004**

(51) Int Cl.:
***A61B 17/70*** (2006.01)

(86) Internationale Anmeldenummer:
**PCT/CH2004/000257**

(87) Internationale Veröffentlichungsnummer:
**WO 2005/104969 (10.11.2005 Gazette 2005/45)**

(54) **VORRICHTUNG ZUR DYNAMISCHEN STABILISIERUNG VON KNOCHEN**

DEVICE FOR DYNAMIC BONE STABILIZATION

DISPOSITIF POUR LA STABILISATION DYNAMIQUE D'OS

(84) Benannte Vertragsstaaten:
**AT CH DE FR GB IT LI**

(43) Veröffentlichungstag der Anmeldung:
**10.01.2007 Patentblatt 2007/02**

(73) Patentinhaber: **Synthes GmbH**
**4436 Oberdorf (CH)**

(72) Erfinder:
• **SCHLÄPFER, Fridolin**
**CH-4434 Hölstein (CH)**

• **HESS, Martin**
**CH-4434 Hölstein (CH)**

(74) Vertreter: **Lusuardi, Werther**
**Dr. Lusuardi AG**
**Kreuzbühlstrasse 8**
**8008 Zürich (CH)**

(56) Entgegenhaltungen:
FR-A1- 2 726 995          US-A1- 2003 171 749
US-A1- 2003 191 470       US-A1- 2004 049 189
US-B1- 6 267 764

EP 1 740 111 B1

**Beschreibung**

Die Erfindung bezieht sich auf eine Vorrichtung zur dynamischen Stabilisierung von Knochen und Knochenfragmenten, insbesondere von Wirbelkörpern, gemäss dem Oberbegriff des Patentanspruchs 1.

Die Wirbelsäule ist das Zentrum eines komplexen Prozesses zur Austarierung von externen und internen Kräften und Momenten. Die Kräfte und Momente werden dabei durch die Muskeln balanciert mit der Wirbelsäule als Widerlager.

Die Wirbelsäule hat gleichzeitig eine formgebende Funktion (Biegen nach vorne und hinten, Ausdrehen, etc.) und spielt auch eine grosse Rolle bei der Dämpfung.

Bei der Stabilisierung von Segmenten der Wirbelsäule auf Grund von degenerativen Erkrankungen, Frakturen, Deformitäten etc. muss darauf geachtet werden, dass der Einfluss der Stabilisierung auf die Funktionen der Wirbelsäule möglichst minimal gehalten wird.

In Fällen, wo keine Korrekturkräfte notwendig sind, kann dies bewerkstelligt werden, indem die betroffen Segmente nicht fusioniert sondern deren geschwächte Strukturen gezielt intern durch ein dynamisches, mit der Wirbelsäule verbundenes System gestützt werden. Dabei muss realisiert werden, dass im Gegensatz zur Bandscheibenendoprothese die betroffenen Strukturen nicht entfernt werden. Dementsprechend löst eine Wiederherstellung des physiologischen Bewegungsmusters je nach Degenerationsgrad nicht unbedingt das klinische Problem.

Hauptindikationen für ein dynamisches System sind Erkrankungen, Entzündungen und/oder Verletzungen im Bereich der Bandscheibe, des Bandapparates, der Fazettengelenke und/oder des subchondralen Knochens. In diesen Situationen ist es wichtig, dass

a) der Schmerz behoben und der Fortgang der Pathologie zumindest eingefroren werden,

b) eine den Metabolismus der Strukturen (Bandscheibe, Bänder, Fazettengelenke) unterstützende Restbewegung übrigbleibt.

Klinisch identifizierbare Schmerzzonen sind von posterior nach anterior gesehen die Fazettengelenke, der posteriore Annulus, und der subchondrale Knochen unterhalb der knöchernen Deckplatte des Wirbelkörpers. Die Schmerzen können rein mechanischer Natur sein oder durch aseptische Entzündungen (durch mechanische und/oder chemische Reizungen bewirkt) ausgelöst werden. Linderung der Schmerzen und Abheilung von Entzündungen bedingen eine Reduktion der Belastung und damit der Dehnung der betroffenen Strukturen.

Der AO Wirbelsäulen Fixateur externe (Systembeschreibung in "Fixateur externe", Autoren: B.G. Weber und F. Magerl, Springer-Verlag 1985, Seite 290 - 366) wird derzeit vor allem für diagnostische Zwecke eingesetzt. Auf Grund seiner Konfiguration ist er sehr steif in Schub und Rotation und flexibel in Flexion/Extension und Seitneigung. Durch die extrem posteriore Lage des Rahmens des Fixateur extern werden bei Flexion und Extension die Bandscheibe und die knöchernen Deckplatten der angrenzenden Wirbeln mehr oder weniger gleichmässig in Kompression belastet. Im Gegensatz dazu findet beim intakten Bewegungssegment ein Abrollen über den zentralen Nucleus statt. Dabei werden der posteriore und anteriore Teil des Annulus wechselseitig auf Zug und Druck belastet. Die grösste Belastung der knöchernen Deckplatten der angrenzenden Wirbeln tritt dabei im Bereich des Nucleus auf. Da das Nucleusmaterial eine gelartige Masse darstellt, wird die Masse bei Flexion und Extension hin- und hergewalkt und dabei gegen den Annulus gepresst. Wenn der Anulus Risse auf Grund eines degenerativen Prozesses oder eines traumatischen Vorfalles aufweist, dringt bei dieser Walkbewegung Nucleusmaterial in die Risse ein. Wenn das Nucleusmaterial dabei bis in den innervierten und vaskularisierten Aussenbereich des Annulus vordringt oder gar aus der Bandscheibe austritt und mit der entsprechenden Nervenwurzel in Kontakt kommt, können entzündliche Prozesse mit den entsprechenden Schmerzen ausgelöst werden. Das aus dem Zusammenspiel zwischen Fixateur externe und Bandscheibe entstandene Bewegungsmuster (bestehend aus einer mehr oder weniger vertikalen Bewegung im Bereich der Bandscheibe) reduziert das Risiko, dass Nucleusmaterial in den Aussenbereich des Annulus gedrückt bzw. gepumpt wird.

Mit dem Ausschliessen der Walkbewegung bei Flexion und Extension werden durch den Fixateur extern gleichzeitig die knöchernen Deckplatten der angrenzenden Wirbel und damit der darunterliegende subchondrale Knochen entlastet. Diese Entlastung kann bei entzündlichen Veränderungen im subchondralen Knochen (beschrieben durch Modic Change 1) zu einer Reduktion der Schmerzen führen. Mit der Entlastung der Deckplatten sind auch die Voraussetzungen für eine Abheilung der Entzündung geschaffen.

Bei dehydriertem Nucleus fehlt der radiale Druck auf den Annulus mit dem Effekt, dass die bei Flexion und Extension entstehende hohe lokale Belastung des Annulus zu dessen Delamination führen kann. Durch den Fixateur externe wird, wie schon oben erwähnt, der bei Flexion und Extension entstehende Druck gleichmässig über die ganze Bandscheibe verteilt. Damit wird das Risiko einer Delamination des posterioren Annulus reduziert.

Die klinische Erfahrung mit dem diagnostisch eingesetzten AO Wirbelsäulen Fixateur externe zeigt, dass ein System, das steif in Rotation und Schub und flexibel in Flexion/Extension und Seitneigung ist, zu einer Reduktion der Beschwerden führen kann.

Da der AO Wirbelsäulen Fixateur externe nur für diagnostische Zwecke und damit nur kurzfristig eingesetzt wird, gibt die mit dem System gewonnene klinische Erfahrung keine Information, inwieweit mit einem System wie dem Fixateur externe der Metabolismus der Weichteilstrukturen aufrecht erhalten bleibt.

Das im Patent EP 0 669 109 B1 (Baumgartner) beschriebene System wird seit einigen Jahren klinisch erfolgreich eingesetzt. Durch die posteriore Lage der Längsträger entsteht eine ähnliche Situation im Bandscheibenbereich wie beim AO Wirbelsäulen Fixateur externe. Die mehrjährige klinische Erfahrung mit dem System deutet darauf hin, dass der Metabolismus der Weichteilstrukturen aufrecht erhalten bleibt.

Basierend auf der klinischen Erfahrung mit dem AO Wirbelsäulen Fixateur interne und dem im Patent EP 0 669 109 B1 (Baumgartner) beschriebenen System muss ein zur Stützung der Wirbelsäulenstrukturen eingesetztes System steif auf Rotation und Schub und flexibel auf Flexion und Extension sein, um einerseits die Beschwerden zu reduzieren und andererseits den Metabolismus der betroffenen Strukturen aufrecht zu erhalten, letzteres um die Voraussetzungen für biologisches Remodeling und Heilung zu schaffen. Ein solches System ist im Oberbegriff des Patentanspruchs 1 beschrieben.

Das im Patent EP 0 669 109 B1 (Baumgartner) beschriebene System verschiebt den Drehpunkt des Wirbelsäulensegmentes aus der Bandscheibe heraus nach posterior in den Bereich der Fazettengelenke. Auf Grund des zentral gelegenen Bandes weist das System eine im Vergleich zum AO Wirbelsäulen Fixateur externe reduzierte Rotations-und Schubsteifigkeit auf.

Neben der reduzierten Rotations- und Schubsteifigkeit hat das im Patent EP 0 669 109 B1 (Baumgartner) beschriebene System auch Nachteile in Bezug auf die Handhabung:

> o Aufwendiges Auffädeln der Stützelemente auf das Band

> o Zuschneiden der Stützelemente auf die gewünschte Länge. Dabei muss darauf geachtet werden, dass die Schnittflächen parallel zu den Seitenflächen der Knochenschrauben zu stehen kommen.

> o Wenn die Knochenschrauben nach anterior divergierend eingebracht sind, ist es schwierig von posterior her die Stützelemente zwischen die Schraubenköpfe zu bringen.

Durch die zentrale Lage des Bandes federt das System nicht automatisch in die Ausgangslage zurück.

Kugelgelenkartige Verbindungen zwischen Pedikelschraube und Längsträger, wie sie zum Beispiel in den Patenten WO 94/00066 (Schläpfer) und PCT/CH02/00672 (Schläpfer) beschrieben werden, erlauben eine spannungsfreie Montage des Implantates mit anschliessender Blockierung aller Freiheitsgrade.

Um trotz der winkelstabilen Verbindung zwischen den Pedikelschrauben und dem Längsträger ein gewisses dynamisches Verhalten des Konstruktes zu gewährleisten, können entweder die beiden oberen (kranialen) Kugelverbindungen zwischen den oberen Pedikelschrauben und den Längsträgern lose gelassen oder die Längsträger selbst flexibel gestaltet werden.

Flexible Längsträger werden unter anderem in den Patenten DE 42 39 716 C1 (Winter), WO 95/27444 (Alby), WO 98/22033 (Elberg), WO 02/102259 A2 (Sengupta) und WO 93/20771 (Mazel) beschrieben. Die beiden erstgenannten Patente umfassen Systeme, die aus beweglichen Teilen bestehen. Bewegliche Teile haben den grossen Nachteil von Abtrieb.

Die beiden Patente WO 98/22033 (Elberg) und WO 02/102259 A2 (Sengupta) beschreiben Systeme mit gebogenen, federnden Längsträgern. Beim erstgenannten Patent weist der Längsträger einen Anschlag auf, der die Biegung in einer Richtung beschränkt. Richtig ausgelegt, ermöglichen die in den beiden Patenten beschriebenen Systeme eine dynamische Stabilisierung der geschwächten Strukturen der Wirbelsäule.

Das Patent WO 93/20771 (Mazel) umschreibt ein System, das aus flexiblen Stäben besteht. Auch hier ist, wenn richtig ausgelegt, eine dynamische Stabilisierung der geschwächten Strukturen der Wirbelsäule möglich.

Eine weitere Vorrichtung zur dynamischen Stabilisierung von Wirbelkörpern ist aus der US-A 2003/0191470 RITLAND bekannt.

Der Erfindung liegt die Aufgabe zu Grunde, einen mit Knochenverankerungsmitteln verbindbaren Längsträger zu schaffen, der bei einer vorgegebenen Festigkeit, insbesondere Schubsteifigkeit eine maximale Flexibilität in Biegung aufweist.

Die Erfindung löst die gestellte Aufgabe mit einer Vorrichtung zur dynamischen Stabilisierung von Knochen und Knochenfragmenten, insbesondere von Wirbelkörpern, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile gegenüber den bestehenden Systemen sind wie folgt:

> o Maximale Biegeflexibilität bei vorgegebener Festigkeit

o Entkopplung von Schubsteifigkeit und Biegeflexibilität

o Eine Kombination mit den in den Patenten WO 94/00066 (Schläpfer) und PCT/CH02/00672 (Schläpfer) beschriebenen, dreidimensionalen Verbindungen zwischen Längsträger und Knochenverankerungsmittel ermöglicht eine spannungsfreie Montage des Längsträgers.

Wenn eine Fusion durchgeführt wird, muss das zur Stabilisierung verwendete System eine so grosse Steifigkeit aufweisen, dass die Bewegung im zu fusionierenden Bewegungssegment so klein ist, dass eine knöcherne Überbrückung der Wirbel stattfindet. Das zur Stabilisierung verwendete System ist mechanisch gesehen parallel zum zu fusionierende Wirbelsäulensegment geschaltet, d.h. je steifer das System ist, um so grösser ist der über das System geleitete Anteil der Wirbelsäulenbelastung. Dementsprechend stark muss das System ausgelegt werden.

Im Gegensatz dazu ist wie oben ausführlich dargelegt bei einer dynamischen Stabilisierung eines Wirbelsäulensegmentes Biegeflexibilität kombiniert mit Kompressions-, Rotations- und Schubsteifigkeit gefragt.

Bei der mechanischen Dimensionierung muss ein dynamisches Fixationssystem mit besagten Eigenschaften dementsprechend in Bezug auf Biegung auf eine Maximaldeformation und in Bezug auf Kompression, Schub und Rotation auf eine Maximallast dimensioniert werden. Die entsprechenden Bedingungen können mit der linearen Elastizitätstheorie angenähert werden.

Im Falle eines von posterior durch die Pedikel verankerten dynamischen Fixationssystems werden die Längsträger vorwiegend in Biegung belastet.

Dementsprechend können die oben dargelegten Bedingungen für die einzelnen als Biegebalken wirkenden Längsträger durch

a) die Differentialgleichung der elastischen Biegelinie der Balkenachse (Eulersche Elastika)

$$\frac{w(z)''}{(1 + w(z)'^2)^{3/2}} = \frac{M_b(z)}{E' * I_y(z)} \tag{1}$$

b) die Momentengleichung

$$M_b(z) = M_y + F_y * (l - z) - F_z * w(z) \tag{2}$$

c) das Hooke'sche Gesetz

$$\sigma_b(x,z) = \frac{M_b(z)}{I_y(z)} * z \tag{3}$$

d) und die Fliessbedingung nach Maxwell (von Mises) oder Tresca

$$\sigma_x^2 + \sigma_z^2 - \sigma_x * \sigma_z + 3 * \tau_{xz}^2 (x,z) \quad \text{von Mises} \tag{4a}$$

$$\sigma_x^2 + \sigma_z^2 - \sigma_x * \sigma_z + 4 * \tau_{xz}^2 (x,z) \quad \text{Tresca} \tag{4b}$$

beschrieben werden.

Bei Variierung der Dicke des Querschnittes und/oder der Materialsteifigkeit (mit oder ohne zusätzlicher Faserverstärkung wie z.B. Kohlefasern) entlang dem Längsträger wird es möglich, Biegeflexibilität mit Schubsteifigkeit zu kombinieren. Die Schubkräfte werden über die kranialen Pedikelschrauben auf das Konstrukt übertragen ; die durch die Schubkräfte im Konstrukt bewirkte Biegebelastung nimmt linear von kranial (Null) nach kaudal (Maximum) zu. Im Gegensatz dazu bewirken in der sagittalen Ebene agierende Biegemomente eine homogene Biegebelastung über die ganze Länge des Konstruktes. Wenn nun die Längsträger des Konstruktes kaudal möglichst steif und kranial möglichst flexibel gestaltet werden, kann das Konstrukt Schubkräfte aufnehmen, während es auf Biegung nach wie vor flexibel ist.

Die einzelnen Längsträger bestehen aus "z" seriell angeordneten Segmenten, wobei $z \geq 3$ ist. Der Uebergang zwischen den einzelnen Segmenten kann kontinuierlich verlaufen oder diskret (abrupt) sein.

Die einzelnen Segmente unterscheiden sich in der Grösse und Form des Querschnittes und/oder der Materialsteifigkeit. Innerhalb der Segmente selbst kann der Querschnitt wie auch die Materialsteifigkeit variieren.

Von den z Segmenten sind mindestens die Endsegmente im Vergleich zu den "n" dazwischen liegenden, flexiblen Segmenten in Biegung mindestens doppelt so steif.

Die "n" flexiblen Segmente mit $1 \leq n \leq z-2$ sind wie schon erwähnt derart ausgelegt, dass sie bei einer vorgegebenen Biegedeformation des Längsträgers nicht mechanisch versagen. Die besagte Biegedeformation des Längsträgers ist die Summe der in den einzelnen flexiblen Segmenten auftretenden Deformation.

Die Querschnittsflächen der n flexiblen Segmente können beispielsweise rechteckig, trapezförmig, rhombisch, dreieckig (aber nicht gleichseitig), oval oder elliptisch sein.

In einer bevorzugten Ausführungsform sind die Querschnittsflächen mindestens eines der n flexiblen Segmente mit einer abgeflachten Querschnittsfläche rechteckig ausgebildet. Der Vorteil dieser Ausgestaltung liegt darin, dass mit einer rechteckigen Gestaltung des Querschnittes der flexiblen Segmente der Längsträger bei einer vorgegebenen Flexibilität eine optimale Festigkeit erreicht wird. Bei Variierung des Querschnittes von kaudal nach kranial (kaudal dick, kranial dünn), kann Biegeflexibilität mit Rotationssteifigkeit und Schubsteifigkeit kombiniert werden. Die anzustrebende, bei einer vorgegebenen Festigkeit maximale Flexibilität in Biegung wird nur durch eine rechteckige Gestaltung des Querschnittes des flexiblen Teils des Längsträgers erreicht. Eine rechteckige Gestaltung des Längsträgers bedingt eine dreidimensionale kugelige Verbindung zwischen Längsträger und Pedikelschraube wie sie zum Beispiel in den Patenten WO 94/00066 (Schläpfer) und PCT/CH02/00672 (Schläpfer) beschrieben ist.

Die $z - n$ im wesentlichen steifen Segmente des Längsträgers sind vorzugsweise zylindrisch oder prismatisch ausgebildet, wobei deren zur Längsachse orthogonale Querschnittsfläche vorzugsweise radialsymmetrisch ausgestaltet sind. Der Längsträger umfasst axial aussenstehend je eines dieser $z - n$ steifen Segmente, so dass der Längsträger mittels dieser aussenstehenden Segmente in den Verbindungsteilen unter verschiedenen - im Falle einer kreiszylindrischen Ausgestaltung dieser aussenstehenden steifen Segmente mit einem beliebigen - Drehwinkel relativ zur Längsachse verbindbar ist. Die Querschnittsfläche dieser $z - n$ steifen Segmente, insbesondere der aussenstehenden steifen Segmente ist vorzugsweise kreisförmig.

Unter der Annahme, dass der Querschnitt und die Materialsteifigkeit des Segmentes über dessen Länge L konstant bleibt, gilt für $n = 1$ flexible Segmente:

$$\text{Dicke} \leq \sigma_{zul} / E \times \text{„freie Länge"} \times 2 / tg\, \alpha$$

$$e \leq \sigma_{zul} / E \times L \times 28 \quad \text{für } \alpha = 4°$$

wobei:

$\alpha$ .... Deformationswinkel des Längsträgers, zwischen 4° und 12°, vorzugsweise 8°

$e$ .... senkrecht zur Biegeachse gemessene Dicke eines elastischen Segmentes

$L$ .... Länge des elastischen Segmentes

$\sigma_{zul}$ .... dynamische Biegefestigkeit des Materials (z.B. 48 N/mm$^2$ für Kunststoff bis 1400 N/mm$^2$ für Stahl)

$E$ .... Elastizitätsmodul (28 N/mm$^2$ für Kunststoff bis 220'000 N/mm$^2$ für Stahl)

$\sigma_{zul} / E$: $0.5 \geq \sigma_{zul} / E \geq 0.005$

Vorzugsweise sind die n flexiblen Segmente mit Abmessungen innerhalb der folgenden Grenzen ausgestaltet:

- Breite b zwischen 8 mm und 15 mm, vorzugsweise zwischen 10 mm und 13 mm;
- Dicke e zwischen 0,5 mm und 3 mm, vozugsweise zwischen 0,9 und 1,1 mm; und
- Länge L zwischen 2 mm und 30 mm, vorzugsweise zwischen 5 mm und 15 mm.

Für n > 1 flexible Segmente gilt:

$$e^i \leq \sigma^i_{zul} / E^i \times L^i \times 2 / tg\, \alpha^i$$

wobei: i = 1 bis n

$$\Sigma^{i=1\ bis\ n}\, \alpha^i = \alpha \qquad 4° \leq \alpha \leq 12°$$

Vorzugsweise sind die n flexiblen Segmente mit Abmessungen innerhalb der folgenden Grenzen ausgestaltet:

- Breite b zwischen 8 mm und 15 mm, vorzugsweise zwischen 10 mm und 13 mm;
- Dicke e zwischen 0,5 mm und 3 mm, vozugsweise zwischen 0,9 und 1,1 mm; und
- Länge L zwischen 2 mm und 30 mm, vorzugsweise zwischen 5 mm und 15 mm.

Der Längsträger wird vorzugsweise aus einem der folgenden Materialien hergestellt:

- aus hochfestem Titan;

- aus einer hochfesten Titanlegierung;

- aus hochfestem Stahl;

- aus einer Cobalt-Chrom Legierung;

- aus einem hochfesten, monokristallinen Material; oder

- aus einem durch hochfeste, monokristallinen Fasern verstärkten Material.

Die Erfindung und Weiterbildungen der Erfindung werden im folgenden anhand der teilweise schematischen Darstellungen mehrerer Ausführungsbeispiele noch näher erläutert.
Es zeigen:

Fig. 1 eine perspektivische Darstellung einer Ausführungsform der erfindungsgemässen Vorrichtung;

Fig. 2 eine zur Längsachse orthogonale Querschnittsfläche eines flexiblen Segmentes des Längsträgers in einer Ausführungsform der erfindungsgemässen Vorrichtung;

Fig. 3 einen Schnitt durch ein Knochenverankerungsmittel gemäss einer Ausführungsform der erfindungsgemässen Vorrichtung;

Fig. 4 eine Ansicht einer Ausführungsform der erfindungsgemässen Vorrichtung als Wirbelsäulenstabilisationsvorrichtung;

Fig. 5a eine schematische Darstellung eines mittels zweier Knochenverankerungsmitel an zwei benachbarten Wirbelkörpern befestigten Längsträgers gemäss einer Ausführungsform der erfindungsgemässen Vorrichtung;

Fig. 5b einen Ausschnitt aus einem flexiblen Segment eines Längsträgers gemäss einer Ausführungsform der erfindungsgemässen Vorrichtung; und

Fig. 6 eine Graphik zur vereinfachten Darstellung der Bruchkriterien für den Längsträger gemäss einer Ausführungsform der efindungsgemässen Vorrichtung.

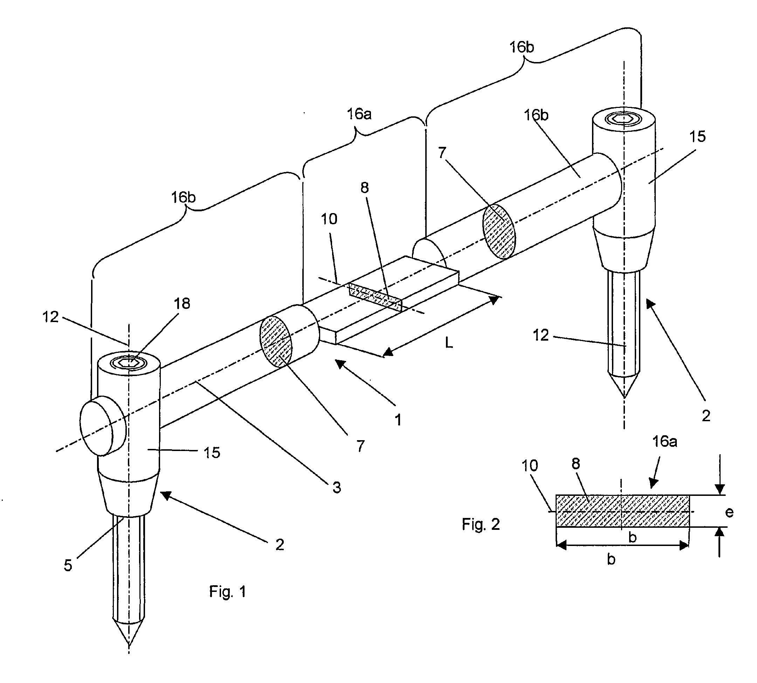

In Fig. 1 ist eine Ausführungsform dargestellt, welche zwei als Pedikelschrauben 5 mit integrierten Verbindungsteilen 15 ausgeführte Knochenverankerungsmittel 2 mit je einer Zentralachse 12 und einen Längsträger 1 mit einer Längsachse 3 umfasst. Der Längsträger 1 besteht aus gesamthaft z = 3 Segmenten 16, wobei die beiden axial aussenstehenden Segmente 16b steif sind und das eine Länge L aufweisende, flexible Segment 16a (n = 1) axial zwischen den zwei steifen Segmenten 16b angeordnet ist. Wie in Fig. 2 gezeigt weist das mittig angeordnete, flexible Segment 16a eine zur Längsachse 3 orthogonale rechteckige Querschnittsfläche 8 mit einer Breite b und einer Dicke e auf. Dabei ist die Breite b der rechteckförmigen Querschnittsfläche 8 quer zu der Längsachse 3 des Längsträgers 1 und quer zu den Zentralachsen 12 der Knochenverankerungsmittel 2 angeordnet. Das flexible Segment 16a ist durch seine Form bevorzugt bezüglich der mit einer zur langen Achse der rechteckförmigen Querschnittsfläche 8 zusammenfallenden oder parallelen Biegeachse 10 elastisch biegbar. Die Knochenverankerungsmittel 2 sind über die Verbindungsteile 15 mit den steifen, kreiszylindrisch ausgebildeten Segmenten 16b des Längsträgers 1 lösbar verbunden.

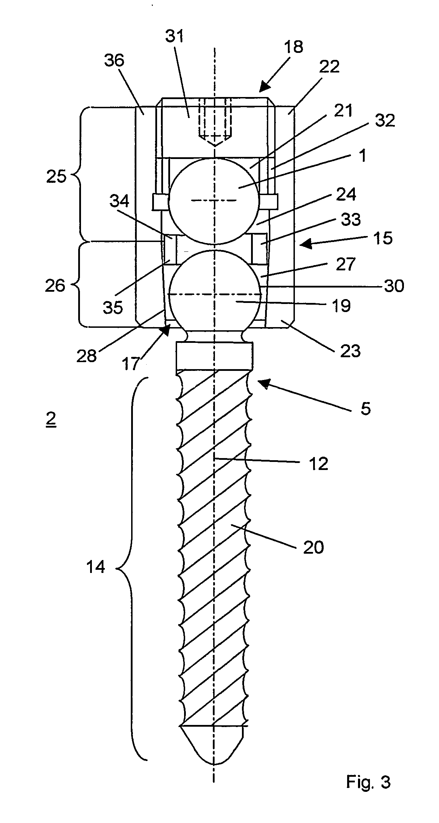

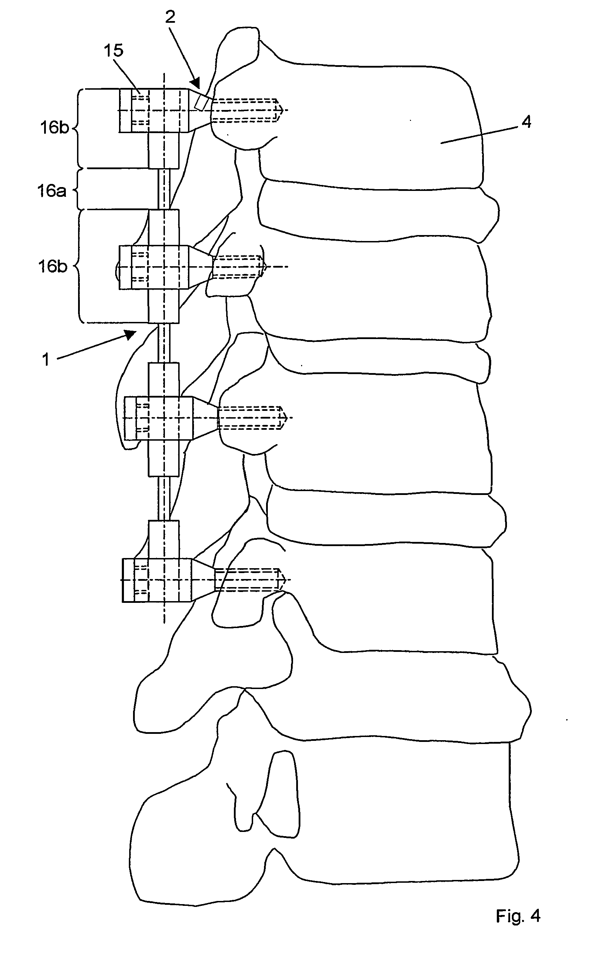

Die in Fig. 3 dargestellte Ausführungsform der Knochenverankerungsmittel 2 umfassen Pedikelschrauben 5, welche je ein in einen Pedikel eines Wirbelkörpers 4 (Fig. 4) einschraubbares Verankerungsteil 14 und je einen kugelförmigen Schraubenkopf 19 umfassen. Der kugelförmige Schraubenkopf 19 bildet die eine Komponente des Kugelgelenkes 17 durch das die Knochenverankerungsmittel 2 polyaxial schwenkbar mit dem Verbindungsteil 15 verbindbar ist.

Diese Pedikelschrauben 5 bestehen aus einem koaxialen Schraubenschaft 20 und einem oben an den Schraubenschaft angrenzenden, konzentrisch angeordneten kugelförmigen Schraubenkopf 19. Das Verbindungsteil 15 ist derart ausgebildet, dass der Längsträger 1 vor seiner Fixation am Knochenverankerungsmittel 2 in einen im Hohlkörper 36 angeordneten Kanal 21 eingelegt und anschliessend mittels der Spannmittel 18 im Kanal 21 fixiert wird. Der Kanal 21 durchdringt den Hohlkörper 36 quer zur Zentralachse 12 und ist am oberen Ende 22 des Hohlkörpers 36 offen.

Der Hohlkörper 36 umfasst ein die Zentralachse 12 schneidendes, oberes Ende 22, ein die Zentralachse 12 schneidendes, unteres Ende 23 und eine Kavität 24, welche hier den Hohlkörper 36 koaxial vom oberen Ende 22 bis zum unteren Ende 23 durchdringt. Die Kavität 24 besteht aus zwei axial hintereinander angeordneten Segmenten 25; 26, wovon das obere Segment 25 eine koaxial zylindrische Bohrung umfasst, in welcher das radial elastisch deformierbare Klemmelement 27 axial verschiebbar gelagert ist, und das untere Segment 26 sich gegen des untere Ende 23 des Verbindungsteils 15 verjüngend konisch ausgebildet ist. Die Aussenwand 28 des Klemmelementes 27 ist zum Innenkonus 29 im unteren Segmentes 26 komplementär konisch ausgestaltet, so dass das Klemmelement 27 - wenn es in der Kavität 24 koaxial gegen das untere Ende 26 des Verbindungsteiles 15 gepresst wird - radial komprimiert wird. Ferner umfasst das Klemmelement 27 einen axial durchgehend offenen Hohlraum 30, welcher hier zum Schraubenkopf 19 komplementär sphärisch ausgebildet ist. Der Schraubenkopf 19 ist im dekomprimierten Zustand des Klemmelementes 27 vom unteren Ende 26 des Verbindungsteiles 15 in den Hohlraum 30 einschnappbar. Im komprimierten Zustand des Klemmelementes 27 wird der Schraubenkopf 19 im Hohlraum 30 blockiert. Wegen des sphärischen Ausgestaltung des Schraubenkopfes 19 und des Hohlraumes 30 ist das Knochenverankerungsmittel 2 relativ zum Verbindungsteil 15 polyaxial schwenkbar und auch unter verschiedenen Winkeln zwischen der Zentralachse 12 des Knochenverankerungsmittels 2 und der Achse des Verbindungsteiles 15 blockierbar.

Die axiale Verschiebung des Klemmelementes 27 erfolgt hier mittels der als Spannschraube 31 ausgebildeten Spannmittel 18, welche in ein zu ihrem Gewinde komplementäres Innengewinde 32 im oberen Segment 25 der Kavität 24 einschraubbar ist. Das vordere Ende der Spannschraube 31 drückt beim Anziehen auf den in den Kanal 21 eingelegten Längsträger 1. Damit beim Anziehen der Spannmittel 18 neben dem Längsträger 1 auch der Schraubenkopf 19 im Verbindungsteil 15 fixierbar ist, ist zwischen dem Längsträger 1 und dem Klemmelement 27 ein ringförmiges Zwischenstück 33 angeordnet. Die Tiefe T des Kanals 21 so gross gewählt, dass der in den Kanal 21 eingelegte Längsträger 1 auf des obere Ende 34 des Zwischenstückes 33 drückt. Das untere Ende 35 des Zwischenstückes 33 liegt auf dem Klemmelement 27 auf. Beim Anziehen der Spannmittel 18 drückt dieses auf den Längsträger 1, wodurch das Zwischenstück 33 zusammen mit dem 21 angrenzenden Klemmelement 27 gegen das untere Ende 23 des Verbindungselementes 15 gepresst wird. Durch das innen konisch ausgebildete untere Segment 26 der Kavität 24 wird das konische Klemmelement 27 radial komprimiert und der Schraubenkopf 19 im Hohlraum 30 des Klemmelementes 27 blockiert.

In Fig. 4 ist eine Anwendung des Längsträgers 1 innerhalb einer Wirbelsäulenstabilisationsvorrichtung dargestellt. Der Längsträger 1 hat z = 7 Segmente 16, wovon n = 3 flexible Segmente 16a und z - n = 4 steife Segmente 16b sind. Je ein steifes Segment 16b ist mit dem Verbindungsteil 15 eines Knochenverankerungsmittels 2 verbunden ist. Dabei ist je ein Knochenverankerungs-mittel 2 in einen Pedikel eines Wirbelkörpers 4 eingeschraubt.

Beschreibung des Implationsvorganges:

• Pedikelschrauben mit polyaxialen Köpfen eindrehen

- Abstand der Pedikelschrauben bestimmen
- Längsträger (vorgebogen) entsprechend den gemessenen Abständen auswählen
- Längsträger einbringen
- Polyaxiale Köpfe der Pedikelschrauben verschliessen
- Je nach Bedarf Komprimieren/Distrahieren der Pedikelschrauben
- Verbindung zwischen Pedikelschraube und Längsträger blockieren

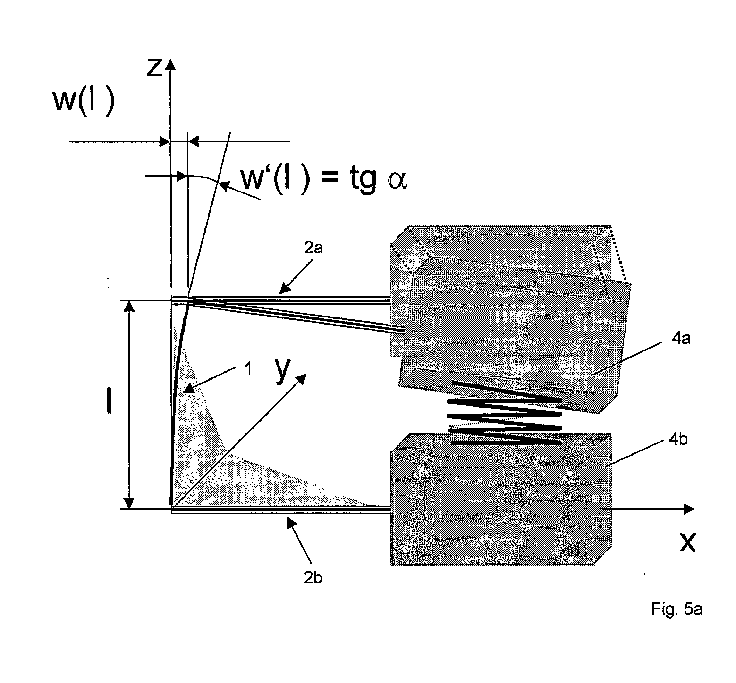

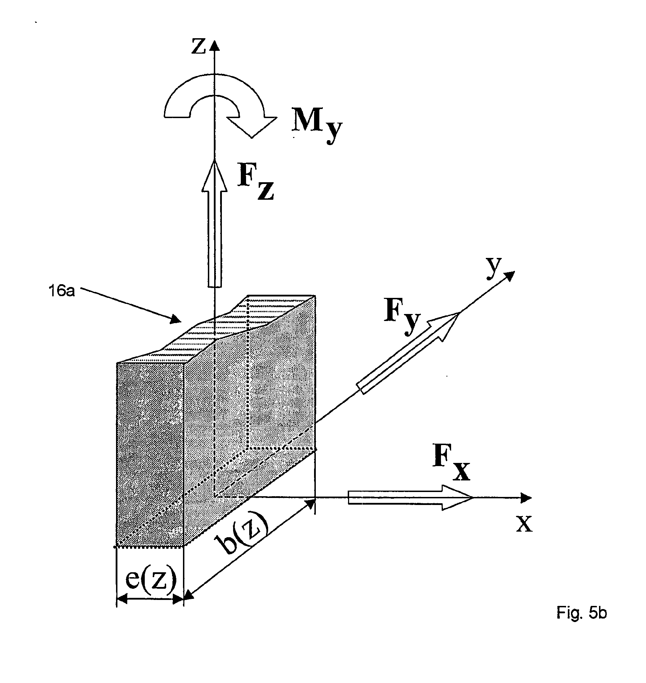

Fig. 5a und 5b illustrieren den durch das nachfolgende Blockdiagramm beschriebenen Zusammenhang zwischen der Deformation des betroffenen Wirbelsäulensegmentes und der Geometrie der Längsträger.

$e(z)_{max}$ repräsentiert die maximal mögliche Dicke der Längsträger, dass bei einer durch die Deformation der Längsträger erzeugte kraniale Bewegung kein Bruch der Längsträger auftritt.

w(l) und w'(l)=tg$\alpha$ sind von Funktionsröntgenbildern und/oder in vitro Tests bekannt.

Da w(l) und w'(l) vom Verhältnis der Steifigkeit des dynamischen Fixationssystems und des überbrückten Wirbelsäulensegmentes abhängt, kann $|e(z)|_{max}$ genau genommen nur iterativ erarbeitet werden. Wenn aber die Biegesteifigkeit des überbrückten Wirbelsäulensegmentes als viel kleiner als die Biegesteifigkeit des überbrückten Wirbelsäulensegmentes angenommen werden kann (was der Fall ist), können w(l) und w'(l) direkt aus Funktionsröntgenbildern von Patienten gemessen werden, die mit einem funktionsähnlichen Fixationssystem versorgt wurden.

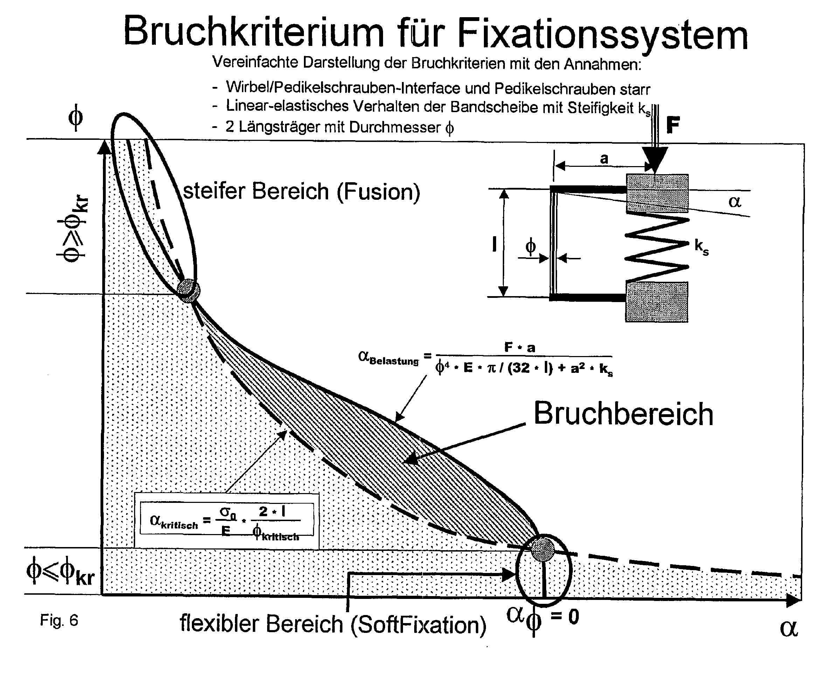

Zusammengefasst kann gesagt werden, dass bei der SoftFixation das Ziel nicht mehr eine Fusion sondern eine funktionelle Abstützung der Strukturen der überbrückten Wirbelsäulensegmente ist. In diesem Zusammenhang muss das Fixationssystem zu einem gewissen Grad mechanisch nachgiebig sein. Dementsprechend muss im Gegensatz zu einer auf eine Fusion hinwirkenden Stabilisierung bei der SoftFixation das Fixationssystem nicht mehr auf eine maximale Belastung sondern auf eine maximale Deformation dimensioniert werden. Wie Figur 6 zeigt, ist ein flexibles System aber erst ab einer gewissen Flexibilität bruchsicher, d.h. das System muss so dimensioniert werden, dass bei einer vorgegebenen Deformation die kritische Flexibilität des Fixationssystems nicht unterschritten wird.

Die in Fig. 6 gezeigte Graphik ist eine vereinfachte Darstellung der Bruchkriterien für ein zur Stabilisierung eines Wirbelsäulensegmentes verwendeten Fixationssystems (oben rechts symbolisch dargestellt).

Die Graphik $\alpha_{kritisch}$ zeigt auf vereinfachte Art den Zusammenhang zwischen der Geometrie des Fixationssystems (symbolisiert durch den Durchmesser $\phi$ der Längsträger) und der maximalen Deformation (symbolisiert durch den Deformationswinkel $\alpha_{kritisch}$), die das System kompensieren kann, bevor es bricht.

Die Graphik zeigt auch, dass mit zunehmender Festigkeit und zunehmender Flexibilität des Materials sich der kritische Bereich nach rechts verschiebt.

Die Graphik $\alpha_{Belastung}$ ist die Kennlinie des Fixationssystems. Sie zeigt den Zusammenhang zwischen Geometrie (symbolisiert durch den Durchmesser $\phi$ der Längsträger) und Deformation des Fixationssystems (symbolisiert durch den Deformationswinkel $\alpha$) für eine vorgegebene Wirbelsäulenbelastung (symbolisiert durch F) in Abhängigkeit der mechanischen Eigenschaften von Fixationssystem (symbolisiert durch Zugfestigkeit $\sigma_0$ und Elastizitätsmodul E) und Wirbelsäulensegment (symbolisiert durch die linear-elastisch Federkonstante $k_s$).

Solange die Kennlinie $\alpha_{Belastung}$ des Systems links von der Graphik $\alpha_{Belastung}$ liegt, ist das Fixationssystem nicht bruchgefährdet.

In der vorliegenden Figur überschneiden sich die Graphiken zweimal. Bei der Dimensionierung eines Fixationssystems bewegte man sich bis anhin im Bereich oberhalb des obern Schnittpunktes der beiden Graphiken.

Das Ziel der Stabilisierung mit einem Fixationssystem war die Fusion der überbrückten Segmente. Wie die Graphik zeigt, darf dabei die Steifigkeit des Systems (unter anderem gegeben durch den Durchmesser der Längsträger) einen bestimmten Wert nicht unterschreiten.

Bei der SoftFixation ist das Ziel nicht mehr eine Fusion, sondern eine funktionelle Abstützung der Strukturen der überbrückten Wirbelsäulensegmente. In diesem Zusammenhang muss das Fixationssystem zu einem gewissen Grad mechanisch nachgiebig sein. Dementsprechend muss im Gegensatz zu einer auf eine Fusion hinwirkenden Stabilisierung bei der SoftFixation das Fixationssystem nicht mehr auf eine maximale Belastung sondern auf eine maximale Deformation dimensioniert werden. Wie die vorliegende Graphik zeigt, ist ein flexibles System erst ab einer gewissen Flexibilität bruchsicher, d.h. das System muss so dimensioniert werden, dass bei einer vorgegebenen Deformation die kritische Flexibilität des Fixationssystems nicht unterschritten wird. Die Erfindung betrifft die Definition dieser Bedingung und deren diverse Realisierungsmöglichkeiten.

**Patentansprüche**

1. Vorrichtung zur dynamischen Stabilisierung von Knochen oder Knochenfragmenten, insbesondere von Wirbelkörpern (4) mit

   A) mindestens einem eine Längsachse (3) aufweisenden longitudinalen Längsträger (1); und
   B) mindestens zwei Knochenverankerungsmitteln (2) mit je einer Zentralachse (12) und je einem zur Verankerung an einem Knochen bestimmten Verankerungsteil (14), wobei
   C) jedes Knochenverankerungsmittel (2) mittels eines mit Spannmitteln (18) ausgestatteten Verbindungsteils (15) an einem Längsträger (1) lösbar befestigbar ist,
   wobei der mindestens eine Längsträger (1) in axialer Richtung
   D) eine Anzahl $n \geq 1$ flexible Segmente (16a) mit einer zur Längsachse (3) orthogonalen Querschnittsfläche (8) umfasst, welche weder einem Kreis noch einem regelmässigen Polygon entspricht, sowie
   E) eine Anzahl $m = (n + 1)$ von im wesentlichen steifen Segmente (16b) beliebiger Querschnittsfläche umfasst, so dass
   F) gesamthaft eine Anzahl $z \geq (2n + 1)$ von axialen Segmenten (16a; 16b) resultiert,
   **dadurch gekennzeichnet, dass**
   G) sich das Elastizitätsmodul des Materials mindestens eines der flexiblen Segmente (16a) entlang der Längsachse (3) verändert.

2. Vorrichtung nach Anspruch 1, **dadurch gekennzeichnet, dass** mindestens eines der flexiblen Segmente (16a) über die gesamte Länge eine konstante Querschnittsfläche (8) aufweist.

3. Vorrichtung nach Anspruch 1, **dadurch gekennzeichnet, dass** die Querschnittsfläche (8) sich bei mindestens einem der flexiblen Segmente (16a) entlang der Längsachse (3) verändert.

4. Vorrichtung nach Anspruch 3, **dadurch gekennzeichnet, dass** die Querschnittsfläche (8) sich kontinuierlich entlang der Längsachse (3) verändert.

5. Vorrichtung nach einem der Ansprüche 1 bis 4, **dadurch gekennzeichnet, dass** $n \geq 2$ ist und das Elastizitätsmodul des Materials mindestens eines der flexiblen Segmente (16a) über die gesamte Länge konstant ist.

6. Vorrichtung nach einem der Ansprüche 1 bis 4, **dadurch gekennzeichnet, dass** das Elastizitätsmodul des Materials sich kontinuierlich entlang der Längsachse (3) verändert.

7. Vorrichtung nach einem der Ansprüchen 1 bis 6, **dadurch gekennzeichnet, dass** die Dicken $e_i$ der flexiblen Segmente (16a) so klein sind, dass

   a) die Summe der Deformationen $\alpha^i$ der einzelnen flexiblen Segmenten (16a) eine Gesamtdeformation $\alpha$ des Längsträgers (3) ergibt, welche 12° nicht überschreitet, und
   b) die in den Segmenten (16) resultierenden maximalen Biegespannungen die zulässige Biegespannung des Materials nicht überschreitet.

8. Vorrichtung nach einem der Ansprüche 1 bis 7, **dadurch gekennzeichnet, dass** die Querschnittsfläche (8) mindestens eines der n flexiblen Segmente (16a) rechteckig ausgebildet sind.

9. Vorrichtung nach einem der Ansprüche 1 bis 8, **dadurch gekennzeichnet, dass** der Längsträger (1) axial aussenstehend je ein steifes Segment (16b) mit einer zur Längsachse (3) orthogonalen, radial symmetrischen Querschnittsfläche (7) umfasst.

10. Vorrichtung nach einem der Ansprüche 1 bis 9, **dadurch gekennzeichnet, dass** der Längsträger (1) $m = (z - n)$ im wesentlichen steife Segmente (16b) mit einer zur Längsachse (3) orthogonalen, radialsymmetrischen Querschnittsfläche (7) umfasst.

11. Vorrichtung nach Anspruch 10, **dadurch gekennzeichnet, dass** die n flexiblen Segmente (16a) und die m im wesentlichen steifen Segmente (16b) alternierend angeordnet sind.

12. Vorrichtung nach einem der Ansprüche 1 bis 11, **dadurch gekennzeichnet, dass** mindestens eines der n flexiblen Segmente (16a) eine Querschnittsfläche (8) mit einer Breite b und einer Dicke e aufweist, wobei das Verhältnis e/b maximal 0,4 beträgt.

13. Vorrichtung nach einem der Ansprüche 1 bis 12, **dadurch gekennzeichnet, dass** mindestens eines der n flexiblen Segmente (16a) eine Querschnittsfläche (8) mit einer Breite b und einer Dicke e aufweist, wobei das Verhältnis e/b mindestens 0,05 beträgt.

14. Vorrichtung nach einem der Ansprüche 1-13,
**dadurch gekennzeichnet, dass**
mindestens eines der n flexiblen Segmente (16a) mit den angrenzenden steifen Segmenten (16b) verbindbar ist.

15. Vorrichtung nach einem der Ansprüche 1 bis 14, **dadurch gekennzeichnet, dass** mindestens eines der n flexiblen Segmente (16a) aus einem biokompatiblen Kunststoff gefertigt ist.

16. Vorrichtung nach einem der Ansprüche 1 bis 14, **dadurch gekennzeichnet, dass** mindestens eines der n flexiblen Segmente (16a) aus einem hochfesten, monokristallinen Material besteht.

17. Vorrichtung nach einem der Ansprüche 1 bis 16, **dadurch gekennzeichnet, dass** mindestens eines der n flexiblen Segmente (16a) durch hochfeste, monokristalline Fasern verstärkt wird.

18. Vorrichtung nach einem der Ansprüche 1 bis 15, **dadurch gekennzeichnet, dass** mindestens eines der n flexiblen Segmente (16a) aus einem faserverstärkten, biokompatiblen Kunststoff besteht.

19. Vorrichtung nach einem der Ansprüche 1 bis 18, **dadurch gekennzeichnet, dass** mindestens ein Verbindungsteil (15) ein Kugelgelenk (17) zur polyaxial schwenkbaren Verbindung des Knochenverankerungsmittels (2) mit dem Längsträger (1) umfasst.

20. Vorrichtung nach einem der Ansprüche 1 bis 7 oder 9 bis 19, **dadurch gekennzeichnet, dass** die Querschnittsfläche (8) mindestens eines der n flexiblen Segmente (16a) oval ausgebildet ist.

21. Vorrichtung nach einem der Ansprüche 1 bis 20, **dadurch gekennzeichnet, dass** mindestens eines der n flexiblen Segmente (16a) hohl ist.

22. Vorrichtung nach Anspruch 21, **dadurch gekennzeichnet, dass** mindestens eines der n flexiblen Segmente (16a) eine sich axial erstreckende Bohrung aufweist.

23. Vorrichtung nach einem der Ansprüche 1 bis 22, **dadurch gekennzeichnet, dass** jedes der n flexiblen Segmente (16a) axial ein erstes und ein zweites, je an ein im wesentlichen steifes Segment (16b) angrenzendes Ende (37; 38) aufweist und dass die Querschnittsfläche (8) mindestens eines der n flexiblen Segmente (16a) gegen mindestens eines der Enden (37;38) in mindestens einer Dimension zunimmt.

24. Vorrichtung nach Anspruch 23, **dadurch gekennzeichnet, dass** die Querschnittsfläche (8) mindestens eines der n flexiblen Segmente (16a) in mindestens einer Dimension linear zunimmt.

25. Vorrichtung nach Anspruch 23, **dadurch gekennzeichnet, dass** die Querschnittsfläche (8) mindestens eines der n flexiblen Segmente (16a) in mindestens einer Dimension progressiv zunimmt.

26. Vorrichtung nach einem der Ansprüche 1 bis 25, **dadurch gekennzeichnet, dass** der Längsträger (1) relativ zu den Knochenverankerungsmitteln (2) derart angeordnet ist, dass die maximale Abmessung der zur Längsachse (3) orthogonalen Querschnittsfläche (8) mindestens eines der n flexiblen Segmente (16a) quer zu den Zentralachsen (12) der Knochenverankerungsmittel (2) angeordnet ist.

**Claims**

1.  Device for the dynamic stabilization of bones or bone fragments, especially of vertebral bodies (4) with

    A) at least one longitudinal carrier (1) having a longitudinal axis (3); and
    B) at least two bone anchoring means (2) with a central axis (12) each and an anchoring part (14) each, intended for anchoring to a bone,
    C) each bone anchoring means (2) being detachably fastenable to a longitudinal carrier (1) by means of a connecting part (15) equipped with clamping means (18),
    whereby the at least one longitudinal carrier (1) comprises in the axial direction
    D) a number n $\geq$ 1 of flexible segments (16a) with a cross-sectional surface (8), which is orthogonal to the longitudinal axis (3) and corresponds neither to a circle nor to a regular polygon, and
    E) a number m = (n + 1) of essentially stiff segments (16b) of any cross-sectional area, so that
    F) a total number z $\geq$ (2n + 1) of axial segments (16a; 16b) results,
    **characterized in that**
    G) the modulus of elasticity of the material of at least one of the flexible segments (16a) changes along the longitudinal axis (3).

2.  The device of claim 1, **characterized in that** at least one of the flexible segments (16a) has a constant cross-sectional area (8) over the whole of its length.

3.  The device of claim 1, **characterized in that** the cross-sectional area (8) changes along the longitudinal axis (3) in the case of at least one of the flexible segments (16a).

4.  The device of claim 3, **characterized in that** the cross-sectional area (8) changes continuously along the longitudinal axis (3).

5.  The device of one of the claims 1 to 4, **characterized in that** n $\geq$ 2 and the modulus of elasticity of the material of at least one of the flexible segments (16a) is constant over the whole of the length.

6.  The device of one of the claims 1 to 4, **characterized in that** the modulus of elasticity of the material changes continuously along the longitudinal axis (3).

7.  The device of one of the claims 1 to 6, **characterized in that** the thickness $e_i$ of the flexible segments (16a) is so small that

    a) the sum of the deformations $\alpha^i$ of the individual flexible segments (16a) results in a total deformation $\alpha$ of the longitudinal carrier (3), which does not exceed 12° and
    b) the maximum bending stresses, resulting in the segments (16), do not exceed the permissible bending stresses of the material.

8.  The device of one of the claims 1 to 7, **characterized in that** the cross-sectional area (8) of at least one of the n flexible segments (16a) is rectangular.

9.  The device of one of the claims 1 to 8, **characterized in that** the longitudinal carrier (1) has at each axial end a stiff segment (16b) with a cross-sectional area (7), which is radially symmetrical and orthogonal to the longitudinal axis (3).

10. The device of one of the claims and 1 to 9, **characterized in that** the longitudinal carrier (1) has m = (z - n) essentially stiff segments (16b) with a cross-sectional area (7) which is radially symmetrical and orthogonal to the longitudinal axis (3).

11. The device of claim 10, **characterized in that** the n flexible segments (16a) and the m essentially stiff segments (16b) alternate with one another.

12. The device of one of the claims 1 to 11, **characterized in that** at least one of the n flexible segments (16a) has a cross-sectional surface (8) with a width b and a thickness e, the ratio of e:b being at most 0.4.

13. The device of one of the claims 1 to 12, **characterized in that** at least one of the n flexible segments (16a) has a cross-sectional surface (8) with a width b and a thickness e, the ratio of e:b being at least 0.05.

14. Device of one of the claims 1 to 13, **characterized in that** at least one of the n flexible segments (16a) can be connected with the adjoining, stiff segments (16b).

15. The device of one of the claims 1 to 14, **characterized in that** at least one of the n flexible segments (16a) is made from a biocompatible plastic.

16. The device of one of the claims 1 to 14, **characterized in that** at least one of the n flexible segments (16a) consists of a high strength, monocrystalline material.

17. The device of one the claims 1 to 16, **characterized in that** at least one of the n flexible segments (16a) is reinforced by high strength, monocrystalline fibers.

18. The device of one of the claims 1 to 15, **characterized in that** at least one of the n flexible segments (16a) consists of a fiber-reinforced, biocompatible plastic.

19. The device of one of the claims 1 to 18, **characterized in that** at least one connecting part (15) comprises a ball joint (17) for the polyaxially pivotable connection of the bone anchoring means (2) to the longitudinal carrier (1).

20. The device of one of the claim 1 to 7 or 9 to 19, **characterized in that** the cross-sectional area (8) of at least one of the n flexible segments (16a) is constructed ovally.

21. The device of one of the claims 1 to 20, **characterized in that** at least one of the n flexible segments (16a) is hollow.

22. The device of claim 21, **characterized in that** at least one of the n flexible segments (16a) has an axially extending borehole.

23. The device of one of the claims 1 to 22, **characterized in that** each of the n flexible segments (16a) axially has a first and a second end (37; 38), each of which adjoins an essentially stiff segment (16b) and that the cross-sectional surface (8) of at least one of the n flexible segments (16a) increases in at least one dimension in the direction of at least one of the ends (37; 38).

24. The device of claim 23, **characterized in that** the cross-sectional area (8) of at least one of the n flexible segments (16a) increases linearly in at least one dimension.

25. The device of claim 23, **characterized in that** the cross-sectional area (8) of at least one of the n flexible segments (16a) increases progressively in at least one dimension.

26. The device of one of the claims 1 to 25, **characterized in that** the longitudinal carrier (1) is disposed in such a manner relative to the bone anchoring means (2), that the maximum dimension of the cross-sectional surface (8) of at least one of the n flexible segments (16a), disposed orthogonally to the longitudinal axis (3), is disposed transversely to the central axes (12) of the bone anchoring means (2).

**Revendications**

1. Dispositif de stabilisation dynamique d'os ou de fragments osseux, notamment de corps vertébraux (4), ledit dispositif comprenant :

   A) au moins un support longitudinal (1) comportant un axe longitudinal (3) ; et
   B) au moins deux moyens (2) d'ancrage à un os qui possèdent chacun un axe central (12) et une pièce d'ancrage (14) destinée à l'ancrage à un os,
   C) chaque moyen (2) d'ancrage à un os pouvant être fixé de façon amovible à un support longitudinal (1) au moyen d'une pièce de liaison (15) dotée de moyens de serrage (18), l'au moins un support longitudinal (1) comportant dans une direction axiale
   D) un nombre n ≥ 1 de segments flexibles (16a) ayant une section (8) qui est orthogonale à l'axe longitudinal (3) et qui ne correspond ni à un cercle ni à un polygone régulier, ainsi que

E) un nombre m = (n + 1) de segments sensiblement rigides (16b) de section quelconque de sorte que

F) l'on obtient dans l'ensemble un nombre z ≥ (2n + 1) de segments axiaux (16a ; 16b),

**caractérisé en ce que**

G) le module d'élasticité du matériau d'au moins un des segments flexibles (16a) varie le long de l'axe longitudinal (3).

2. Dispositif selon la revendication 1, **caractérisé en ce qu'**au moins un des segments flexibles (16a) a une section constante (8) sur toute la longueur.

3. Dispositif selon la revendication 1, **caractérisé en ce que** la section (8) varie, pour au moins un des segments flexibles (16a), le long de l'axe longitudinal (3).

4. Dispositif selon la revendication 3, **caractérisé en ce que** la section (8) varie de façon continue le long de l'axe longitudinal (3).

5. Dispositif selon l'une des revendications 1 à 4, **caractérisé en ce que** n ≥ 2 et le module d'élasticité du matériau d'au moins un des éléments flexibles (16a) est constant sur toute la longueur.

6. Dispositif selon l'une des revendications 1 à 4, **caractérisé en ce que** le module d'élasticité du matériau varie de façon continue le long de l'axe longitudinal (3).

7. Dispositif selon l'une des revendications 1 à 6, **caractérisé en ce que** les épaisseurs $e_i$ des segments flexibles (16a) sont telles que

a) la somme des déformations $\alpha^i$ des segments flexibles individuels (16a) donne une déformation totale $\alpha$ du support longitudinal (3) qui n'est pas supérieure à 12°, et

b) les contraintes de flexion maximales générées dans les segments (16) ne sont pas supérieures à la contrainte de flexion admissible du matériau.

8. Dispositif selon l'une des revendications 1 à 7, **caractérisé en ce que** la section (8) d'au moins un des n segments flexibles (16a) a une conformation rectangulaire.

9. Dispositif selon l'une des revendications 1 à 8, **caractérisé en ce que** le support longitudinal (1) comporte, axialement à chaque bout, un segment rigide (16b) ayant une section (7) radialement symétrique et orthogonale à l'axe longitudinal (3).

10. Dispositif selon l'une des revendications 1 à 9, **caractérisé en ce que** le support longitudinal (1) possède m = (z - n) segments sensiblement rigides (16b) ayant une section (7) radialement symétrique et orthogonale à l'axe longitudinal (3).

11. Dispositif selon la revendication 10, **caractérisé en ce que** les n segments flexibles (16a) et les m segments sensiblement rigides (16b) sont disposés de façon alternée.

12. Dispositif selon l'une des revendications 1 à 11, **caractérisé en ce qu'**au moins un des n segments flexibles (16a) a une section (8) de largeur b et d'épaisseur e, le rapport e/b ayant une valeur maximale de 0,4.

13. Dispositif selon l'une des revendications 1 à 12, **caractérisé en ce qu'**au moins un des n segments flexibles (16a) a une section (8) de largeur b et d'épaisseur e, le rapport e/b ayant une valeur minimale de 0,05.

14. Dispositif selon l'une des revendications 1 à 13, **caractérisé en ce qu'**au moins un des n segments flexibles (16a) peut être relié aux segments rigides adjacents (16b).

15. Dispositif selon l'une des revendications 1 à 14, **caractérisé en ce qu'**au moins un des n segments flexibles (16a) est fabriqué à partir d'une matière plastique biocompatible.

16. Dispositif selon l'une des revendications 1 à 14, **caractérisé en ce qu'**au moins un des n segments flexibles (16a) est en un matériau monocristallin très résistant.

**17.** Dispositif selon l'une des revendications 1 à 16, **caractérisé en ce qu'**au moins un des n segments flexibles (16a) est renforcé par des fibres monocristallines très résistantes.

**18.** Dispositif selon l'une des revendications 1 à 15, **caractérisé en ce qu'**au moins un des n segments flexibles (16a) est en une matière plastique biocompatible renforcée par des fibres.

**19.** Dispositif selon l'une des revendications 1 à 18, **caractérisé en ce qu'**au moins une pièce de liaison (15) comporte une articulation sphérique (17) pour relier, avec un pivotement polyaxial, le moyen (2) d'ancrage à un os au support longitudinal (1).

**20.** Dispositif selon l'une des revendications 1 à 7 ou 9 à 19, **caractérisé en ce que** la section (8) d'au moins un des n segments flexibles (16a) a une conformation ovale.

**21.** Dispositif selon l'une des revendications 1 à 20, **caractérisé en ce qu'**au moins un des n segments flexibles (16a) est creux.

**22.** Dispositif selon la revendication 21, **caractérisé en ce qu'**au moins un des n segments flexibles (16a) comporte un perçage s'étendant axialement.

**23.** Dispositif selon l'une des revendications 1 à 22, **caractérisé en ce que** chacun des n segments flexibles (16a) comporte des première et seconde extrémités (37 ; 38) chacune adjacentes à un segment sensiblement rigide (16b), et **en ce que** la section (8) d'au moins un des n segments flexibles (16a) augmente dans au moins une dimension en direction d'au moins une des extrémités (37 ; 38).

**24.** Dispositif selon la revendication 23, **caractérisé en ce que** la section (8) d'au moins un des n segments flexibles (16a) augmente linéairement dans au moins une dimension.

**25.** Dispositif selon la revendication 23, **caractérisé en ce que** la section (8) d'au moins un des n segments flexibles (16a) augmente progressivement dans au moins une dimension.

**26.** Dispositif selon l'une des revendications 1 à 25, **caractérisé en ce que** le support longitudinal (1) est disposé par rapport aux moyens (2) d'ancrage à un os de telle sorte que la dimension maximale de la section (8), orthogonale à l'axe longitudinal (3), d'au moins un des n segments flexibles (16a) est disposé transversalement aux axes centraux (12) des moyens (2) d'ancrage à un os.

Fig. 1

Fig. 2

Fig. 3

Fig. 4

Fig. 5a

Fig. 5b

# Bruchkriterium für Fixationssystem

Vereinfachte Darstellung der Bruchkriterien mit den Annahmen:

- Wirbel/Pedikelschrauben-Interface und Pedikelschrauben starr
- Linear-elastisches Verhalten der Bandscheibe mit Steifigkeit $k_s$
- 2 Längsträger mit Durchmesser $\phi$

$\phi$

$\phi \gg \phi_{kr}$

steifer Bereich (Fusion)

**F**

$a$

$\alpha$

$l$ $\phi$ $k_s$

$$\alpha_{Belastung} = \frac{F \cdot a}{\phi^4 \cdot E \cdot \pi / (32 \cdot l) + a^2 \cdot k_s}$$

Bruchbereich

$$\alpha_{kritisch} = \frac{\sigma_0}{E} \cdot \frac{2 \cdot l}{\phi_{kritisch}}$$

$\phi \leqslant \phi_{kr}$

flexibler Bereich (SoftFixation) $\alpha_{\phi} = 0$

Fig. 6

$\alpha$

EP 1 740 111 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0669109 B1, Baumgartner
- WO 9400066 A, Schläpfer
- CH 0200672 W, Schläpfer
- DE 4239716 C1, Winter
- WO 9527444 A, Alby
- WO 9822033 A, Elberg
- WO 02102259 A2, Sengupta
- WO 9320771 A, Mazel
- US 20030191470 A

**In der Beschreibung aufgeführte Nicht-Patentliteratur**

- **B.G. Weber ; F. Magerl.** Systembeschreibung in ''Fixateur externe''. Springer-Verlag, 1985, 290-366